(19) Europäisches Patentamt
European Patent Office
Office européen des brevets

(11) EP 3 922 311 A1

(12) EUROPEAN PATENT APPLICATION
published in accordance with Art. 153(4) EPC

(43) Date of publication:
15.12.2021 Bulletin 2021/50

(51) Int Cl.:
A61P 17/00 (2006.01)
A61K 9/12 (2006.01)
A61K 47/14 (2017.01)
A61K 9/08 (2006.01)
A61K 47/02 (2006.01)
A61K 47/32 (2006.01)

(21) Application number: 20752808.4

(22) Date of filing: 03.02.2020

(86) International application number:
PCT/JP2020/003947

(87) International publication number:
WO 2020/162402 (13.08.2020 Gazette 2020/33)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 04.02.2019 PCT/JP2019/003868

(71) Applicant: Maruho Co., Ltd.
Osaka 531-0071 (JP)

(72) Inventors:
• NAKAMURA Ayako
Osaka-shi, Osaka 531-0071 (JP)
• SAKIYAMA Hiroki
Osaka-shi, Osaka 531-0071 (JP)
• HAFSI Leila
Osaka-shi, Osaka 531-0071 (JP)
• OTSUKA Naoya
Osaka-shi, Osaka 531-0071 (JP)

(74) Representative: Pfenning, Meinig & Partner mbB
Patent- und Rechtsanwälte
Theresienhöhe 11a
80339 München (DE)

(54) SKIN COMPOSITION

(57) It is an object of the present invention to provide a film-forming composition for skin (such as a film-type skin protectant or a film-type skin topical agent) that does not contain a lower monohydric alcohol such as ethanol or isopropanol, is excellent in quick-drying properties and water resistance, is less sticky, and is excellent in feeling of use. The present invention relates to a film-forming composition for skin which contains a specific acrylic polymer, a plasticizer and water and is substantially free of a lower monohydric alcohol such as ethanol or isopropanol. The composition of the present invention is extremely useful for treatment of chronic skin diseases (that is, skin diseases in which skin barrier function is deteriorated) represented by hand eczema, atopic dermatitis, and the like.

FIGURE 1

EP 3 922 311 A1

**Description**

TECHNICAL FIELD

The present invention relates to a film-forming composition, specifically, a film-forming skin protectant and a film-forming skin topical agent.

BACKGROUND ART

A liquid adhesive bandage that forms a thin film is considered to be effective for symptoms in which the skin barrier function is deteriorated (hangnail, abrasion, cracking, etc.), and is commercially available for the purpose of protecting skin from kitchen work or the like. However, many of them contain a lower monohydric alcohol such as ethanol or isopropanol in order to enhance quick-drying properties, and have irritation at the time of application. Similarly, in hand eczema, asteatosis, atopic dermatitis and the like in which skin barrier function is deteriorated, affected area often extends over a wider range, and it is difficult to use a film-forming composition containing a lower monohydric alcohol that brings about dryness and irritation of skin. It is desired from patients and medical personnel to develop a film-forming composition that does not contain these lower monohydric alcohols (that is, skin safety is high), in which a film is maintained even by contact with water (that is, protective effect is sustained).

Patent Documents 1 and 2 disclose a topical agent for fingers that forms a soft film when applied to entire fingers, is excellent in quick-drying properties and adhesion to the skin, and can be gently washed off without giving irritation to the skin after use. However, this topical agent for fingers contains a lower monohydric alcohol such as ethanol in terms of quick-drying properties and the like.

PRIOR ART DOCUMENTS

PATENT DOCUMENTS

Patent Document 1: JP-A-2011-126796
Patent Document 2: JP-A-2011-126797

SUMMARY OF THE INVENTION

PROBLEMS TO BE SOLVED BY THE INVENTION

Therefore, an object of the present invention is to provide a film-forming composition for skin that is excellent in quick-drying properties and water resistance, is less sticky, and is excellent in feeling of use even without containing a lower monohydric alcohol such as ethanol or isopropanol, specifically, a film-forming skin protectant and a film-forming skin topical agent.

MEANS FOR SOLVING THE PROBLEMS

As a result of repeated studies to solve the above problems, the present inventors have found that the above problems can be solved by a combination of a specific acrylic polymer and a plasticizer, and have completed the present invention.

The present invention relates to the following [1] to [10].

[1] A film-forming composition for skin, wherein the composition contains:

(a) one or more acrylic polymers selected from the group consisting of an ethyl acrylate-methyl methacrylate-trimethylammonioethyl methacrylate chloride copolymer, an ethyl acrylate-methyl methacrylate copolymer, a methyl acrylate-methyl methacrylate-methacrylic acid copolymer, and a methacrylic acid-ethyl acrylate copolymer;
(b) one or more plasticizers selected from the group consisting of an ester compound that is liquid at ordinary temperature, an aromatic alcohol that is liquid at ordinary temperature, a medium-polar solid ester compound, and a terpene; and
(c) 36% by weight or more of water based on the total amount of the composition (provided that a propellant is excluded when the composition is an aerosol), and

the composition is substantially free of a lower monohydric alcohol.
[2] The composition according to [1], wherein

the ester compound that is liquid at ordinary temperature is selected from triethyl citrate, triacetin, dibutyl phthalate, diethyl sebacate, diisopropyl sebacate, diisopropyl adipate, and medium-chain triglycerides,
the aromatic alcohol that is liquid at ordinary temperature is selected from ethylene glycol salicylate and phenoxyethanol,
the medium-polar solid ester compound is selected from a phospholipid and a parahydroxybenzoic acid ester, and
the terpene is selected from limonene and menthol.

[3] The composition according to [1], containing at least one plasticizer selected from triethyl citrate, triacetin, dibutyl phthalate, diethyl sebacate, diisopropyl sebacate, diisopropyl adipate, and medium-chain triglycerides.
[4] The composition according to [1], containing at least one plasticizer selected from ethylene glycol salicylate and phenoxyethanol.
[5] The composition according to [1], containing at least one plasticizer selected from a phospholipid and a parahydroxybenzoic acid ester.
[6] The composition according to [1], containing at least one plasticizer selected from limonene and menthol.
[7] The composition according to any one of [1] to [6], wherein the content of the acrylic polymer is 6 to 35% by weight based on the total amount of the composition (provided that a propellant is excluded when the composition is an aerosol).
[8] The composition according to any one of [1] to [7], wherein the content of the plasticizer is 1.3 to 35% by weight based on the total amount of the composition (provided that a propellant is excluded when the composition is an aerosol).
[9] The composition according to any one of [1] to [8], further containing a propellant.
[10] The composition according to any one of [1] to [9], further containing light anhydrous silicic acid and/or titanium oxide.

EFFECT OF THE INVENTION

Since the composition of the present invention is substantially free of a lower monohydric alcohol such as ethanol or isopropanol, the composition has less skin irritation. In addition, since the composition of the present invention contains a large amount (36% by weight or more) of water, it has a fresh (non-sticky) feeling of use. Further, although the composition of the present invention does not contain a lower monohydric alcohol as described above and contains a large amount of water, the composition is excellent in quick-drying properties. Furthermore, a formed film has water resistance, is less sticky, and surprisingly has an excellent effect of suppressing moisture evaporation. The composition of the present invention can be used as a film-forming skin protectant and a film-forming skin topical agent.

BRIEF DESCRIPTION OF THE DRAWINGS

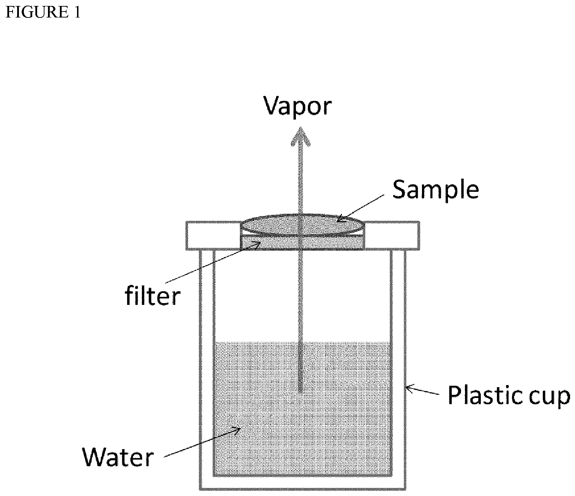

Fig. 1 is a diagram for explaining a water evaporation suppression evaluation test.

MODE FOR CARRYING OUT THE INVENTION

The composition of the present invention contains a specific acrylic polymer, a plasticizer and water, and is substantially free of a lower monohydric alcohol such as ethanol or isopropanol. The composition of the present invention can be specifically used as a film-forming skin protectant and a film-forming skin topical agent.
The film-forming skin protectant (not containing a medicinal ingredient) can form a thin film by being sprayed or applied onto skin to effectively protect the skin, and can be used for prevention or treatment of skin diseases such as hand eczema typified by housewife eczema, asteatosis, and atopic dermatitis.
On the other hand, a film-forming skin topical agent (including a medicinal ingredient) forms a film by being sprayed or applied onto skin, and the medicinal ingredient can be effectively delivered into the skin. In addition, the same effect can be expected when the composition is applied to a nail or a skin around the nail or a mucous membrane. This is formulated in a dosage form such as liquid, lotion, gel, cream or aerosol, and can be applied to an antibacterial agent, an antifungal agent, a vitamin agent, an anti-inflammatory agent, an antiallergic agent, a moisturizing agent, a wound therapeutic agent, and the like.

The acrylic polymer used in the present invention is preferably an ethyl acrylate-methyl methacrylate-trimethylammonioethyl methacrylate chloride copolymer, an ethyl acrylate-methyl methacrylate copolymer, a methyl acrylate-methyl methacrylate-methacrylic acid copolymer, or a methacrylic acid-ethyl acrylate copolymer. These polymers are conventionally used as film coating substrates for pharmaceuticals and are known as EUDRAGIT (registered trademark) RS type, EUDRAGIT RL type, EUDRAGIT NE type, EUDRAGIT FS type, and Kollicoat (registered trademark) MAE type.

The acrylic polymer used in the present invention is a water-insoluble acrylic polymer. In the present specification, the water-insoluble acrylic polymer is used to mean not only an acrylic polymer (pH-independent water-insoluble acrylic polymer) that is not dissolved in water regardless of the pH of water but also a pH-dependent water-insoluble acrylic polymer. The pH-dependent water-insoluble acrylic polymer is preferably not dissolved in water at acidic pH (at a pH below 7, especially at a pH below 5.5).

EUDRAGIT RS type is an ethyl acrylate-methyl methacrylate-trimethylammonioethyl methacrylate chloride copolymer, and the composition ratio thereof is 1 : 2 : 0.1. EUDRAGIT RS type has an average molecular weight (Mw) of about 32,000 and is pH-independent water-insoluble.

EUDRAGIT RL type is an ethyl acrylate-methyl methacrylate-trimethylammonioethyl methacrylate chloride copolymer, and the composition ratio thereof is 1 : 2 : 0.2. EUDRAGIT RL type has an average molecular weight (Mw) of about 32,000 and is pH-independent water-insoluble.

EUDRAGIT NE type is an ethyl acrylate-methyl methacrylate copolymer, and the composition ratio thereof is 2 : 1. EUDRAGIT NE type has an average molecular weight (Mw) of about 750,000 and is pH-independent water-insoluble.

EUDRAGIT FS type is a methyl acrylate-methyl methacrylate-methacrylic acid copolymer, and the composition ratio thereof is X : Y : 1 (where X + Y = 10). EUDRAGIT FS type has an average molecular weight (Mw) of about 280,000 and is insoluble in water at a pH below 7.

Kollicoat MAE type is a methacrylic acid-ethyl acrylate copolymer, and the composition ratio thereof is 1 : 1. Kollicoat MAE type has an average molecular weight (Mw) of about 250,000 and is insoluble in water at a pH below 5.5.

EUDRAGIT RS type is available, for example, as EUDRAGIT RS100, EUDRAGIT RSPO, and EUDRAGIT RS30D (an aqueous suspension containing 30% by weight of ethyl acrylate-methyl methacrylate-trimethylammonioethyl methacrylate chloride copolymer (composition ratio 1 : 2 : 0.1)) manufactured by EVONIK (Germany).

EUDRAGIT RL type is available, for example, as EUDRAGIT RL100, EUDRAGIT RLPO, and EUDRAGIT RL30D (an aqueous suspension containing 30% by weight of ethyl acrylate-methyl methacrylate-trimethylammonioethyl methacrylate chloride copolymer (composition ratio 1 : 2 : 0.2)) manufactured by EVONIK (Germany).

EUDRAGIT NE type is available, for example, as EUDRAGIT NE30D (an aqueous suspension containing 30% by weight of ethyl acrylate-methyl methacrylate copolymer (composition ratio 2 : 1)) manufactured by EVONIK (Germany).

EUDRAGIT FS type is available, for example, as EUDRAGIT FS30D (an aqueous suspension containing 30% by weight of methyl acrylate-methyl methacrylate-methacrylic acid copolymer (composition ratio X : Y : 1, where X + Y = 10)) manufactured by EVONIK (Germany).

Kollicoat MAE type is available, for example, as Kollicoat MAE 30 DP (an aqueous suspension containing 30% by weight of methacrylic acid-ethyl acrylate copolymer (composition ratio 1 : 1)) manufactured by BASF (Germany).

The content of the polymer based on the total amount of the composition of the present invention (provided that a propellant is excluded when the composition is an aerosol) is preferably 6 to 35% by weight, more preferably 10 to 30% by weight, and particularly preferably 14 to 28% by weight.

The acrylic polymer used in the present invention may be used alone or in combination. Since the composition of the present invention is excellent in water resistance, it need not particularly contain a silicone-based polymer.

The composition of the invention is substantially free of lower monohydric alcohols (monohydric alcohols having 1 to 3 carbon atoms, for example, ethanol, isopropanol, and the like). Substantially free means that a lower monohydric alcohol is not intentionally added in the production process. Therefore, the lower monohydric alcohol content of the composition of the present invention is usually 0% by weight, and even if it is mixed very slightly, the content is less than 1% by weight (more preferably less than 0.5% by weight).

Usually, a lower monohydric alcohol such as ethanol or isopropanol is used to dissolve an acrylic polymer as a film-forming component. However, when used for treatment of a skin disease with deteriorated barrier function such as hand eczema or atopic dermatitis, it is preferable not to contain a lower monohydric alcohol causing skin dryness or irritation. Since the composition of the present invention does not substantially contain a lower monohydric alcohol, the composition has high safety to the skin, and is suitable for treating hand eczema and atopic dermatitis.

Examples of the plasticizer used in the present invention include an ester compound that is liquid at ordinary temperature (25°C; the same applies hereinafter), an aromatic alcohol that is liquid at ordinary temperature, a medium-polar solid ester compound, and a terpene. These may be used singly or in combination of two or more types thereof.

Preferred examples of the ester compound that is liquid at ordinary temperature include triethyl citrate, triacetin, dibutyl phthalate, diethyl sebacate, diisopropyl sebacate, diisopropyl adipate, and medium-chain triglycerides (for example, glycerin triisooctanoate, tri(caprylic/capric acid) glycerin, and the like).

Fatty acid esters which are selected from diethyl sebacate, diisopropyl sebacate, diisopropyl adipate and medium-chain triglycerides and are liquid at ordinary temperature are more preferable, and among them, diethyl sebacate and diisopropyl adipate are further preferable, and diisopropyl adipate is particularly preferable.

Preferred examples of the aromatic alcohol that is liquid at ordinary temperature include ethylene glycol salicylate and phenoxyethanol.

Examples of the medium-polarity solid ester compound include ester compounds that are solid at ordinary temperature, such as phospholipid (for example, lecithin, especially hydrogenated lecithin) and parahydroxybenzoic acid ester. In the present specification, the medium polarity means that $\alpha$ value according to an organic conceptual diagram is 35° to 55°. The organic conceptual diagram was proposed by Atsushi Fujita, and details thereof are described in "Pharmaceutical Bulletin", 1954, vol. 2, 2, pp. 163 to 173; "Chemistry region", 1957, vol. 11, 10, pp. 719 to 725; "Fragrance journal", 1981, vol. 50, pp. 79 to 82, and the like. That is, a source of all the organic compounds is methane ($CH_4$), and all the other compounds are regarded as derivatives of methane, and certain numerical values are set for a carbon number, substituent, modification part and ring thereof and the like, and the scores are added to determine an organic value and an inorganic value. An angle of inclination when the values are plotted on a diagram in which the organic value is taken on an X-axis and the inorganic value is taken on a Y-axis is the $\alpha$ value.

Examples of combined use of an ester compound that is liquid at ordinary temperature include a combination of a medium-chain triglyceride (for example, glycerin triisooctanoate, tri(caprylic/capric acid) glycerin, and the like), and triethyl citrate, triacetin, dibutyl phthalate, diethyl sebacate, diisopropyl sebacate or diisopropyl adipate.

Examples of combined use of an ester compound that is liquid at ordinary temperature and an aromatic alcohol that is liquid at ordinary temperature include a combination of a medium-chain triglyceride (for example, glycerin triisooctanoate, tri(caprylic/capric acid) glycerin, and the like), and ethylene glycol salicylate or phenoxyethanol.

The content of the plasticizer based on the total amount of the composition of the present invention (provided that a propellant is excluded when the composition is an aerosol) is preferably 1.3 to 35% by weight, more preferably 1.4 to 29% by weight, and particularly preferably 1.4 to 8% by weight. When the amount of the plasticizer is less than 1.3% by weight, film formability and water resistance are deteriorated, and when the amount is more than 35% by weight, the film tends to be sticky, which is not preferable.

The water used in the present invention is particularly preferably purified water. The content of water based on the total amount of the composition of the present invention (provided that a propellant is excluded when the composition is an aerosol) is preferably 36% by weight or more, and more preferably 50% by weight or more. The upper limit is preferably 93% by weight. More specifically, the content is preferably 36 to 92% by weight, more preferably 43 to 89% by weight, particularly preferably 50 to 87% by weight, and further preferably 64 to 85% by weight. EUDRAGIT RS30D, RL30D, NE30D and FS30D, and Kollicoat MAE30DP are all provided as aqueous suspensions containing 30% by weight of an acrylic polymer. Water contained in the aqueous suspensions (corresponding to 70% by weight of the aqueous suspensions) also corresponds to the water used in the present invention.

The composition of the present invention can contain, for example, light anhydrous silicic acid and titanium oxide in order to suppress gloss of the film. The content of the substances based on the total amount of the composition of the present invention (provided that a propellant is excluded when the composition is an aerosol) is preferably 0.8 to 3% by weight, and particularly preferably 1 to 3% by weight.

The composition of the present invention can contain a pH adjusting agent in addition to the above components. Examples of the pH adjusting agent include phosphate, citrate, hydroxide, hydrochloric acid, and the like. The pH adjusting agent may be used singly or in combination of two or more types thereof. The content of the pH adjusting agent based on the total amount of the composition of the present invention (provided that a propellant is excluded when the composition is an aerosol) is preferably 0.01 to 1% by weight, and particularly preferably 0.1 to 0.5% by weight.

When a pH-dependent water-insoluble acrylic polymer is used as the acrylic polymer, it is preferable to adjust the pH of the composition to a pH at which the acrylic polymer is not dissolved.

For example, when EUDRAGIT FS type is used, the pH of the composition is preferably adjusted below 7, and when Kollicoat MAE type is used, the pH of the composition is preferably adjusted below 5.5.

The composition of the present invention can contain, for example, a medicinal ingredient effective for treatment of various skin diseases including chronic skin diseases represented by atopic dermatitis.

Examples of the medicinal ingredient include, but are not limited to, the following ingredients.

- Steroidal anti-inflammatory drugs such as hydrocortisone, dexamethasone, clobetasol 17-propionate, dexamethasone 17-valerate, fluocinonide, halcinonide, amcinonide, difluprednate, and betamethasone butyrate propionate;
- Non-steroidal anti-inflammatory drugs such as indomethacin, ketoprofen, flurbiprofen, felbinac, piroxicam, ibuprofen piconol, bendazac, butyl flufenamate, and bufexamac;
- Anti-fungal drugs such as lanoconazole, tolnaftate, clotrimazole, bifonazole, miconazole nitrate, econazole nitrate, ketoconazole nitrate, omoconazole nitrate, oxiconazole nitrate, exalamide, tricyclate, and siccanin;
- Antimicrobials such as ozenoxacin and clindamycin;
- Anti-allergic drugs such as ketotifen, azelastine and salts thereof, chlorpheniramine maleate, oxatomide, tranilast, and sodium cromoglycate;
- Local anesthetics such as lidocaine and propitocaine;
- Humectants such as heparinoid, hyaluronic acid, and urea;
- Wound therapeutic agents such as fibroblast growth factor and bucladesine sodium;
- Therapeutic agents for acne, such as benzoyl peroxide and adapalene;
- Therapeutic agents for keratosis including psoriasis vulgaris such as maxacalcitol

The content of the medicinal ingredient based on the total amount of the composition of the present invention (provided that a propellant is excluded when the composition is an aerosol) is preferably 0.01 to 5% by weight, more preferably 0.1 to 1% by weight, and particularly preferably 0.2 to 0.5% by weight.

The composition of the present invention may be an aerosol containing a propellant. Examples of the propellant include dimethyl ether (DME) and a mixture of DME and liquefied natural gas (LPG). More preferred examples of the propellant include DME.

The content of the propellant in the aerosol of the present invention is preferably 30 to 50 parts by weight, more preferably 35 to 50 parts by weight, and particularly preferably 40 to 46 parts by weight, based on 100 parts by weight of the composition excluding the propellant.

The composition of the present invention is useful for the treatment of skin diseases such as hand eczema, asteatosis, and atopic dermatitis. Application amount and application frequency of the composition according to the present invention to the skin may be appropriately adjusted according to skin symptoms, concentration of drug in the composition, age of patient, and the like. Usually, one to several applications per day are appropriate.

Although preferred compound names of essential components and optional components used in the composition of the present invention have been described above, the composition of the present invention also includes compositions obtained by arbitrarily combining these components and compositions obtained by arbitrarily combining the concentration ranges of the respective components. In addition, the numerical ranges such as the concentration can be arbitrarily combined, and when a plurality of numerical ranges is described, the upper limit value or the lower limit value of each numerical range can also be arbitrarily combined.

Hereinafter, the present invention will be described in more detail with reference to examples, but the present invention is not limited to examples.

<Preparation Method>

[Formulation Examples 1 and 2]

An ethyl acrylate-methyl methacrylate-trimethylammonioethyl methacrylate chloride copolymer (composition ratio 1 : 2 : 0.2) and purified water were weighed, ethylene glycol salicylate was added thereto, and the mixture was stirred until it became uniform.

[Formulation Examples 3 to 8]

An ethyl acrylate-methyl methacrylate-trimethylammonioethyl methacrylate chloride copolymer (composition ratio 1 : 2 : 0.1) and purified water were weighed, diethyl sebacate was added thereto, and the mixture was stirred until it became uniform. For Formulation Example 3, the same operation was performed without adding diethyl sebacate.

[Formulation Examples 9 to 15]

An ethyl acrylate-methyl methacrylate-trimethylammonioethyl methacrylate chloride copolymer (composition ratio 1 : 2 : 0.1), a medium-chain triglyceride and purified water were weighed, triethyl citrate, triacetin, diisopropyl adipate, diisopropyl sebacate, diethyl sebacate, ethylene glycol salicylate or phenoxyethanol was added thereto, and the mixture was stirred until it became uniform. The resulting mixture was placed in a pressure-resistant container, and DME was press-fitted into the container to obtain an aerosol.

[Formulation Examples 16 to 18]

An ethyl acrylate-methyl methacrylate-trimethylammonioethyl methacrylate chloride copolymer (composition ratio 1 : 2 : 0.1) or an ethyl acrylate-methyl methacrylate copolymer (composition ratio 2 : 1) and purified water were weighed, diethyl sebacate was added thereto, and the mixture was stirred until it became uniform.

[Formulation Examples 19 to 21]

An ethyl acrylate-methyl methacrylate-trimethylammonioethyl methacrylate chloride copolymer (composition ratio 1 : 2 : 0.1) and purified water were weighed, diethyl sebacate and light anhydrous silicic acid were added thereto, and the mixture was stirred until it became uniform. The resulting mixture was placed in a pressure-resistant container, and DME was press-fitted into the container to obtain an aerosol. For Example 19, the same operation was performed without adding light anhydrous silicic acid.

[Formulation Examples 22 to 55]

Formulation Examples shown in Table 7 to Table 12 were prepared using each acrylic polymer according to the preparation method of Formulation Examples 1 to 21 described above. When a pH-dependent water-insoluble acrylic polymer was used as the acrylic polymer, the pH of the composition was adjusted to a range in which the acrylic polymer was insoluble.

Compositions of Formulation Examples are shown in Tables 1 to 12.

<Test Examples>

[Test Example 1] Evaluation of Quick-Drying Properties (Film-Forming Properties)

The compositions of Formulation Examples 1 to 18 and 22 to 55 were applied to a glass slide at 30°C, the state of film after 2 minutes and 5 minutes was visually observed, and quick-drying properties were evaluated according to the following criteria.

<Evaluation criteria>

| Sign | Description |
|------|-------------|
| ++ | Film is formed within 2 minutes after application |
| + | Film is not formed within 2 minutes after application, but film is formed within 5 minutes |
| - | Film is not formed within 5 minutes after application |

[Test Example 2] Evaluation of Water Resistance

The compositions of Formulation Examples 1 to 18 and 22 to 55 were applied to a glass slide at 30°C to form a film. Thereafter, the lower half of the glass slide on which the film was formed was infiltrated with water, the state of film after 1 minute and 10 minutes was visually observed, and water resistance was evaluated according to the following criteria.

<Evaluation criteria>

| Sign | Description |
|------|-------------|
| ++ | Film does not peel off even after 10 minutes after infiltration with water |
| + | Film does not peel off after 1 minute after infiltration with water, but film peels off within 10 minutes |
| - | Film peels off within 1 minute after infiltration with water |

[Test Example 3] Evaluation of Stickiness

The compositions of Formulation Examples 1 to 18 and 22 to 55 were applied to a glass slide at 30°C to form a film. Thereafter, another glass slide was pressed against a film-formed surface of the glass slide , and stickiness (adhesiveness) was evaluated according to the following criteria.

<Evaluation criteria>

| Sign | Description |
|------|-------------|
| ++ | No stickiness is observed on film |
| + | Almost no stickiness is observed on film |
| - | Stickiness is observed on film |

[Test Example 4] Evaluation of Gloss

The compositions of Formulation Examples 19 to 21 were applied to artificial leather (76 × 26 mm) at room temperature, and a film was formed at 30°C. Gloss was evaluated according to the following criteria. The results are shown in Table 5.

<Evaluation criteria>

| Sign | Description |
|------|-------------|
| ++ | No gloss is observed on film |
| + | Almost no gloss is observed on film |
| - | Gloss is observed on film |

[Test Example 5] Evaluation of Water Evaporation Suppression Ability

A circle having a diameter of 4 cm was opened at the center of a plastic cup lid, and a carrier membrane was attached to this portion (see Fig. 1). The lid was placed on a cup containing 50 g of water, and then 0.1 g of the composition of Formulation Example 19 was applied to the carrier membrane. As a control for comparison, a carrier membrane without application of a formulation was used. As the carrier membrane, a membrane filter (pore size: 10 μm, diameter: 47 mm) made of hydrophobic polytetrafluoroethylene manufactured by Millipore was used.

After 4 hours at 30°C/40% RH, the amount of water evaporated from the cup of the formulation application group through the carrier membrane was defined as $WL_x$ (g/h), the amount of water evaporated from the cup of the formulation non-application group through the carrier membrane was defined as $WL_0$ (g/h), and water evaporation suppression rate (%), which is a value showing an occlusive effect, was calculated using the following formula.

$$\text{Water evaporation suppression rate (\%)} = \frac{(WL_0 - WL_X)}{WL_0} \times 100$$

As shown in Table 1, by using a specific acrylic polymer and a specific plasticizer, Formulation Examples 1 and 2 which quickly formed a film (excellent in quick-drying properties) even without containing a lower monohydric alcohol, are excellent in water resistance, are not (or less) sticky, and are excellent in feeling of use were obtained.

[Table 1]

Table 1

| Component | Formulation Example 1 | Formulation Example 2 |
|-----------|----------------------|----------------------|
| Ethyl acrylate-methyl methacrylate - trimethylammonioethyl methacrylate chloride copolymer (composition ratio 1 : 2 : 0.2) | 7.07 | 7.07 |
| Ethylene glycol salicylate | 1.43 | 3.57 |

(continued)

| Component | Formulation Example 1 | Formulation Example 2 |
|---|---|---|
| Purified water | 91.50 | 89.36 |
| Total amount | 100 | 100 |
| Quick-drying properties | + | + |
| Water resistance | ++ | ++ |
| Stickiness | ++ | + |
| (Unit: part by weight) | | |

As shown in Table 2, it has been revealed that even when diethyl sebacate, which is a fatty acid ester that is liquid at ordinary temperature, is used as a plasticizer, a desired formulation that forms a film quickly, is excellent in water resistance, is not (or less) sticky and is excellent in feeling of use can be obtained.

[Table 2]

Table 2

| Component | Formulation Example 3 | Formulation Example 4 | Formulation Example 5 | Formulation Example 6 | Formulation Example 7 | Formulation Example 8 |
|---|---|---|---|---|---|---|
| Ethyl acrylate-methyl methacry late-trimethylammonioethyl methacrylate chloride copolymer (composition ratio 1 : 2 : 0.1) | 14.28 | 14.28 | 14.28 | 14.28 | 14.28 | 14.28 |
| Diethyl sebacate | - | 1.43 | 3.57 | 7.14 | 14.29 | 28.57 |
| Purified water | 85.72 | 84.29 | 82.15 | 78.58 | 71.43 | 57.15 |
| Total amount | 100 | 100 | 100 | 100 | 100 | 100 |
| Quick-drying properties | - | ++ | ++ | ++ | ++ | ++ |
| Water resistance | - | ++ | ++ | ++ | ++ | ++ |
| Stickiness | ++ | ++ | ++ | ++ | + | + |
| (Unit: part by weight) | | | | | | |

In addition to diethyl sebacate, it was examined whether a desired film was obtained for an ester compound that is liquid at ordinary temperature. As shown in Table 3, it has been revealed that even when a medium-chain triglyceride is used as a basic component and combined with an ester compound that is liquid at ordinary temperature or an aromatic alcohol which is liquid at ordinary temperature, a desired formulation which forms a film quickly, is excellent in water resistance, is less sticky and is excellent in feeling of use can be obtained.

[Table 3]

Table 3

| Component | Formulation Example 9 | Formulation Example 10 | Formulation Example 11 | Formulation Example 12 | Formulation Example 13 | Formulation Example 14 | Formulation Example 15 |
|---|---|---|---|---|---|---|---|
| Ethyl acrylate-methyl methacrylate - trimethylammonioethyl methacrylate chloride copolymer (composition ratio 1 : 2 : 0.1) | 14.29 | 14.29 | 14.29 | 14.29 | 14.29 | 14.29 | 14.29 |
| Medium-chain triglyceride | 7.14 | 7.14 | 7.14 | 7.14 | 7.14 | 7.14 | 7.14 |
| Triethyl citrate | 7.14 | — | — | — | — | — | — |
| Triacetin | — | 7.14 | - | — | — | — | — |
| Diethyl sebacate | — | — | — | — | 7.14 | — | — |
| Diisopropyl adipate | — | — | 7.14 | — | — | - | — |
| Diisopropyl sebacate | — | — | — | 7.14 | — | — | — |
| Ethylene glycol salicylate | — | — | — | — | — | 7.14 | — |
| Phenoxyethanol | — | — | — | — | — | — | 2.86 |
| Purified water | 71.43 | 71.43 | 71.43 | 71.43 | 71.43 | 71.43 | 75.71 |
| DME | 42.86 | 42.86 | 42.86 | 42.86 | 42.86 | 42.86 | 42.86 |
| Total amount | 142.86 | 142.86 | 142.86 | 142.86 | 142.86 | 142.86 | 142.86 |
| Quick-drying properties | ++ | ++ | ++ | ++ | ++ | ++ | ++ |
| Water resistance | ++ | ++ | ++ | ++ | ++ | ++ | ++ |
| Stickiness | + | + | + | + | + | + | + |
| (Unit: part by weight) | | | | | | | |

It was examined whether a desired film was similarly obtained even when the amount of an ethyl acrylate-methyl methacrylate-trimethylammonioethyl methacrylate chloride copolymer (composition ratio 1 : 2 : 0.1) was increased or decreased as a water-insoluble acrylic polymer. As shown in Table 4, Formulation Examples 16 and 17 formed a film quickly, were excellent in water resistance, were not (or less) sticky, and were excellent in feeling of use.

Furthermore, it was examined whether a desired film was similarly obtained even with a new ethyl acrylate-methyl methacrylate copolymer (composition ratio 2 : 1) as a water-insoluble acrylic polymer. As shown in Table 4, Formulation Example 18 using an ethyl acrylate-methyl methacrylate copolymer (composition ratio 2 : 1) quickly formed a film, was excellent in water resistance, was not sticky, and was excellent in feeling of use.

[Table 4]

Table 4

| Component | Formulation Example 16 | Formulation Example 6 | Formulation Example 17 | Formulation Example 18 |
|---|---|---|---|---|
| Ethyl acrylate-methyl methacry late-trimethylammonioethyl methacrylate chloride copolymer (composition ratio 1 : 2 : 0.1) | 7.07 | 14.28 | 27.86 | |
| Ethyl acrylate-methyl methacrylate copolymer (composition ratio 2 : 1) | - | - | - | 27.86 |
| Diethyl sebacate | 7.14 | 7.14 | 7.14 | 7.14 |
| Purified water | 85.79 | 78.58 | 65.00 | 65.00 |
| Total amount | 100 | 100 | 100 | 100 |
| Quick-drying properties | ++ | ++ | ++ | ++ |
| Water resistance | ++ | ++ | ++ | ++ |
| Stickiness | + | ++ | ++ | ++ |
| (Unit: part by weight) | | | | |

In general, it is preferable that the topical agent is not conspicuous in appearance at the application site after application of the topical agent. Gloss was considered as one of the factors conspicuous in appearance. Therefore, examinations were made to further reduce the gloss of the film. As shown in Table 5, it was revealed that in Formulation Examples 20 and 21 containing light anhydrous silicic acid, gloss was completely suppressed.

[Table 5]

Table 5

| Component | Formulation Example 19 | Formulation Example 20 | Formulation Example 21 |
|---|---|---|---|
| Ethyl acrylate-methyl methacry late-trimethylammonioethyl methacrylate chloride copolymer (composition ratio 1 : 2 : 0.1) | 14.29 | 14.29 | 14.29 |
| Diethyl sebacate | 7.14 | 7.14 | 7.14 |
| Light anhydrous silicic acid | - | 1.43 | 2.86 |
| Purified water | 78.57 | 77.14 | 75.71 |
| DME | 42.86 | 42.86 | 42.86 |
| Total amount | 142.86 | 142.86 | 142.86 |

(continued)

| Component | Formulation Example 19 | Formulation Example 20 | Formulation Example 21 |
|---|---|---|---|
| Gloss | - | ++ | ++ |
| (Unit: part by weight) | | | |

As shown in Table 6, the water evaporation suppression rate (%) of Formulation Example 19 surprisingly showed a high value of 48.4%. The water evaporation suppression rate is one of indices for evaluating the occlusive effect. In skin diseases such as hand eczema, asteatosis and atopic dermatitis, skin barrier function is deteriorated, and the amount of moisture evaporated from the skin is generally large. By application of the present composition, it is expected that the occlusive effect on the skin is obtained, and moisture evaporation from the skin is suppressed, thereby preventing aggravation of symptoms of the above diseases.

[Table 6]

Table 6

| Component | Formulation Example 19 |
|---|---|
| Ethyl acrylate-methyl methacry late-trimethylammonioethyl methacrylate chloride copolymer (composition ratio 1 : 2 : 0.1) | 14.29 |
| Diethyl sebacate | 7.14 |
| Purified water | 78.57 |
| DME | 42.86 |
| Total amount | 142.86 |
| Water evaporation suppression rate (%) | 48.4 |
| (Unit: part by weight) | |

It was examined whether a desired film was similarly obtained even when the amounts of the acrylic polymer (ethyl acrylate-methyl methacrylate-trimethylammonioethyl methacrylate chloride copolymer) and the plasticizer (diethyl sebacate) were increased or decreased. As shown in Table 7, Formulation Examples 22 to 29 quickly formed a film, were excellent in water resistance, were not (or less) sticky, and were excellent in feeling of use.

[Table 7]

Table 7

| Component | Formulation Example 22 | Formulation Example 23 | Formulation Example 24 | Formulation Example 25 | Formulation Example 26 | Formulation Example 27 | Formulation Example 28 | Formulation Example 29 |
|---|---|---|---|---|---|---|---|---|
| Ethyl acrylate-methyl methacry late-trimethylammonioethyl methacrylate chloride copolymer (composition ratio 1 : 2 : 0.1) | 6.00 | 6.00 | 6.00 | 19.50 | 19.50 | 22.50 | 25.71 | 29.61 |
| Diethyl sebacate | 1.30 | 20.00 | 25.00 | 20.00 | 25.00 | 25.00 | 14.29 | 1.30 |
| Purified water | 92.70 | 74.00 | 69.00 | 60.50 | 55.50 | 52.50 | 60.00 | 69.09 |
| Total amount | 100 | 100 | 100 | 100 | 100 | 100 | 100 | 100 |
| Quick-drying properties | ++ | ++ | ++ | ++ | ++ | ++ | ++ | ++ |
| Water resistance | ++ | ++ | ++ | ++ | ++ | ++ | ++ | ++ |
| Stickiness | ++ | + | + | + | + | + | + | ++ |
| (Unit: part by weight) | | | | | | | | |

EP 3 922 311 A1

It was examined whether a desired film was similarly obtained even when the amounts of another acrylic polymer (ethyl acrylate-methyl methacrylate copolymer) and the plasticizer (diethyl sebacate) were increased or decreased. As shown in Table 8, Formulation Examples 30 to 33 quickly formed a film, were excellent in water resistance, were not (or less) sticky, and were excellent in feeling of use.

[Table 8]

Table 8

| Component | Formulation Example 30 | Formulation Example 31 | Formulation Example 32 | Formulation Example 33 |
|---|---|---|---|---|
| Ethyl acrylate-methyl methacrylate copolymer (composition ratio 2 : 1) | 7.07 | 14.28 | 19.50 | 19.50 |
| Diethyl sebacate | 7.14 | 7.14 | 14.29 | 25.00 |
| Purified water | 85.79 | 78.58 | 66.21 | 55.50 |
| Total amount | 100 | 100 | 100 | 100 |
| Quick-drying properties | ++ | ++ | ++ | ++ |
| Water resistance | ++ | ++ | ++ | ++ |
| Stickiness | ++ | + | + | + |
| (Unit: part by weight) | | | | |

It was examined whether a desired film was similarly obtained even when the amounts of still another acrylic polymer (methacrylic acid-ethyl acrylate copolymer) and the plasticizer (diethyl sebacate) were increased or decreased. As shown in Table 9, Formulation Examples 34 to 38 quickly formed a film, were excellent in water resistance, were not (or less) sticky, and were excellent in feeling of use.

[Table 9]

Table 9

| Component | Formulation Example 34 | Formulation Example 35 | Formulation Example 36 | Formulation Example 37 | Formulation Example 38 |
|---|---|---|---|---|---|
| Methacrylic acid-ethyl acrylate copolymer (composition ratio 1 : 1) | 7.07 | 14.28 | 27.86 | 19.50 | 19.50 |
| Diethyl sebacate | 7.14 | 7.14 | 7.14 | 14.29 | 25.00 |
| Purified water | 85.79 | 78.58 | 65.00 | 66.21 | 55.50 |
| Total amount | 100 | 100 | 100 | 100 | 100 |
| Quick-drying properties | ++ | ++ | ++ | ++ | ++ |
| Water resistance | ++ | ++ | ++ | ++ | ++ |
| Stickiness | ++ | ++ | ++ | + | + |
| (Unit: part by weight) | | | | | |

It was examined whether a desired film was similarly obtained even when triethyl citrate was used as a plasticizer and the amount of the acrylic polymer (methyl acrylate-methyl methacrylate-methacrylic acid copolymer) was increased or decreased. As shown in Table 10, Formulation Example 39 to 41 quickly formed a film, were excellent in water resistance, were not sticky, and were excellent in feeling of use.

[Table 10]

Table 10

| Component | Formulation Example 39 | Formulation Example 40 | Formulation Example 41 |
|---|---|---|---|
| Methyl acrylate-methyl methacrylate-methacrylic acid copolymer (composition ratio X : Y : 1, where X + Y = 10) | 7.07 | 14.28 | 27.86 |
| Triethyl citrate | 3.57 | 3.57 | 3.57 |
| Purified water | 89.36 | 82.15 | 68.57 |
| Total amount | 100 | 100 | 100 |
| Quick-drying properties | ++ | ++ | ++ |
| Water resistance | ++ | ++ | ++ |
| Stickiness | ++ | ++ | ++ |
| (Unit: part by weight) | | | |

It was examined whether a desired film was similarly obtained even when the type and/or amount of the plasticizer was changed and/or the amount of the acrylic polymer (ethyl acrylate-methyl methacrylate-trimethylammonioethyl methacrylate chloride copolymer) was increased or decreased. As shown in Tables 11 and 12, Formulation Examples 42 to 55 quickly formed a film, were excellent in water resistance, were not (or less) sticky, and were excellent in feeling of use.

[Table 11]

Table 11

| Component | Formulation Example 42 | Formulation Example 43 | Formulation Example 44 | Formulation Example 45 | Formulation Example 46 | Formulation Example 47 |
|---|---|---|---|---|---|---|
| Ethyl acrylate-methyl methacrylate - trimethylammonioethyl methacrylate chloride copolymer (composition ratio 1 : 2 : 0.1) | 14.28 | 14.28 | 14.28 | 14.28 | 19.50 | 19.50 |
| Diisopropyl adipate | 7.14 | 3.57 | 1.30 | — | — | — |
| Dibutyl phthalate | — | — | — | 7.14 | 17.00 | — |
| Triethyl citrate | — | — | — | — | — | 17.00 |
| Purified water | 78.58 | 82.15 | 84.42 | 78.58 | 63.50 | 63.50 |
| Total amount | 100 | 100 | 100 | 100 | 100 | 100 |
| Quick-drying properties | ++ | ++ | ++ | ++ | ++ | ++ |
| Water resistance | ++ | ++ | ++ | ++ | ++ | ++ |
| Stickiness | + | ++ | ++ | ++ | + | + |
| (Unit: part by weight) | | | | | | |

EP 3 922 311 A1

18

[Table 12]

Table 12

| Component | Formulation Example 48 | Formulation Example 49 | Formulation Example 50 | Formulation Example 51 | Formulation Example 52 | Formulation Example 53 | Formulation Example 54 | Formulation Example 55 |
|---|---|---|---|---|---|---|---|---|
| Ethyl acrylate-methyl methacrylate - trimethylammonioethyl methacrylate chloride copolymer (composition ratio 1:2:0.1) | 14.28 | 19.50 | 14.28 | 14.28 | 14.28 | 19.50 | 14.28 | 14.28 |
| Ethylene glycol salicylate | 10.00 | 17.00 | — | — | — | — | — | — |
| Phenoxyethanol | — | — | — | — | — | — | — | 1.3 |
| Phospholipid (hydrogenated lecithin) | — | — | 7.14 | — | — | — | — | — |
| Propyl paraoxybenzoate | — | — | — | 1.43 | — | — | — | — |
| 1-Menthol | — | — | — | — | 3.57 | 17.00 | — | — |
| Limonene | — | — | — | — | — | — | 3.57 | — |
| Purified water | 75.72 | 63.50 | 78.58 | 84.29 | 82.15 | 63.50 | 82.15 | 84.42 |
| Total amount | 100 | 100 | 100 | 100 | 100 | 100 | 100 | 100 |
| Quick-drying properties | ++ | ++ | ++ | ++ | ++ | ++ | ++ | ++ |
| Water resistance | ++ | ++ | ++ | ++ | ++ | ++ | ++ | ++ |
| Stickiness | + | + | ++ | ++ | ++ | + | ++ | + |
| (Unit: part by weight) | | | | | | | | |

EP 3 922 311 A1

As a result of the above tests, although the composition of the present invention did not contain a lower monohydric alcohol such as ethanol or isopropanol, the composition quickly formed a film, was excellent in water resistance, was not (or less) sticky, and was excellent in feeling of use.

In conventional film-forming skin compositions, a lower monohydric alcohol such as ethanol or isopropanol has been generally used in terms of dissolution of an acrylic polymer as a film-forming component, quick-drying properties (film-forming properties), and the like. In the present invention, elimination of a lower monohydric alcohol has been attempted in terms of skin safety, and as a result of intensive studies, it has been found that a composition for skin excellent in skin safety, which forms a good film in a short time after application even without containing a lower monohydric alcohol. In addition, since the composition of the present invention contains a large amount of water, the composition has a fresh (non-sticky) feeling of use. Further, it was surprisingly confirmed that the formed film had excellent water evaporation suppression ability. The composition of the present invention having an excellent water evaporation suppression ability is extremely useful for treatment of chronic skin diseases (that is, skin diseases in which skin barrier function is deteriorated) represented by hand eczema, atopic dermatitis, and the like.

**Claims**

1.  A film-forming composition for skin, wherein the composition comprises:

    (a) one or more acrylic polymers selected from the group consisting of an ethyl acrylate-methyl methacrylate-trimethylammonioethyl methacrylate chloride copolymer, an ethyl acrylate-methyl methacrylate copolymer, a methyl acrylate-methyl methacrylate-methacrylic acid copolymer, and a methacrylic acid-ethyl acrylate copolymer;
    (b) one or more plasticizers selected from the group consisting of an ester compound that is liquid at ordinary temperature, an aromatic alcohol that is liquid at ordinary temperature, a medium-polar solid ester compound, and a terpene; and
    (c) 36% by weight or more of water based on the total amount of the composition (provided that a propellant is excluded when the composition is an aerosol), and

    the composition is substantially free of a lower monohydric alcohol.

2.  The composition according to claim 1, wherein

    the ester compound that is liquid at ordinary temperature is selected from triethyl citrate, triacetin, dibutyl phthalate, diethyl sebacate, diisopropyl sebacate, diisopropyl adipate, and medium-chain triglycerides,
    the aromatic alcohol that is liquid at ordinary temperature is selected from ethylene glycol salicylate and phenoxyethanol,
    the medium-polar solid ester compound is selected from a phospholipid and a parahydroxybenzoic acid ester, and
    the terpene is selected from limonene and menthol.

3.  The composition according to claim 1, comprising at least one plasticizer selected from triethyl citrate, triacetin, dibutyl phthalate, diethyl sebacate, diisopropyl sebacate, diisopropyl adipate, and medium-chain triglycerides.

4.  The composition according to claim 1, comprising at least one plasticizer selected from ethylene glycol salicylate and phenoxyethanol.

5.  The composition according to claim 1, comprising at least one plasticizer selected from a phospholipid and a parahydroxybenzoic acid ester.

6.  The composition according to claim 1, comprising at least one plasticizer selected from limonene and menthol.

7.  The composition according to any one of claims 1 to 6, wherein the content of the acrylic polymer is 6 to 35% by weight based on the total amount of the composition (provided that a propellant is excluded when the composition is an aerosol).

8.  The composition according to any one of claims 1 to 7, wherein the content of the plasticizer is 1.3 to 35% by weight based on the total amount of the composition (provided that a propellant is excluded when the composition is an aerosol).

9. The composition according to any one of claims 1 to 8, further comprising a propellant.

10. The composition according to any one of claims 1 to 9, further comprising light anhydrous silicic acid and/or titanium oxide.

FIGURE 1

## INTERNATIONAL SEARCH REPORT

| International application No. |
| --- |
| PCT/JP2020/003947 |

A.  CLASSIFICATION OF SUBJECT MATTER
A61P 17/00(2006.01)i; A61K 9/08(2006.01)i; A61K 9/12(2006.01)i; A61K
47/02(2006.01)i; A61K 47/14(2006.01)i; A61K 47/32(2006.01)i
FI: A61K47/32; A61K9/08; A61K9/12; A61K47/02; A61K47/14; A61P17/00
According to International Patent Classification (IPC) or to both national classification and IPC

B.  FIELDS SEARCHED

Minimum documentation searched (classification system followed by classification symbols)
A61P17/00; A61K9/08; A61K9/12; A61K47/02; A61K47/14; A61K47/32

Documentation searched other than minimum documentation to the extent that such documents are included in the fields searched
| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN); Japio-GPG/FX

C.  DOCUMENTS CONSIDERED TO BE RELEVANT

| Category* | Citation of document, with indication, where appropriate, of the relevant passages | Relevant to claim No. |
| --- | --- | --- |
| A | JP 2015-521995 A (LEO PHARMA A/S) 03.08.2015 (2015-08-03) claims, paragraphs [0034], [0035], [0042], [0051], examples 1, 2 | 1-10 |
| A | JP 03-077820 A (HISAMITSU PHARMACEUTICAL CO., INC.) 03.04.1991 (1991-04-03) claims, page 1, right column, page 2, upper right column, page 2, lower right column, examples 4, 7-10 | 1-10 |
| A | JP 05-032535 A (ZENYAKU KOGYO KABUSHIKI KAISHA) 09.02.1993 (1993-02-09) claims, paragraphs [0008], [0010], [0012], each example | 1-10 |
| A | JP 2004-067539 A (HISAMITSU PHARMACEUTICAL CO., INC.) 04.03.2004 (2004-03-04) paragraph [0029] | 1-10 |
| A | JP 2006-523662 A (AXCESS LIMITED) 19.10.2006 (2006-10-19) paragraph [0002] | 1-10 |

☒  Further documents are listed in the continuation of Box C.    ☒  See patent family annex.

| | |
| --- | --- |
| *  Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| 23 March 2020 (23.03.2020) | 31 March 2020 (31.03.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**INTERNATIONAL SEARCH REPORT**

| International application No. |
| --- |
| PCT/JP2020/003947 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2006-160610 A (SHISEIDO CO., LTD.) 22.06.2006 (2006-06-22) paragraph [0005] | 1-10 |
| A | JP 2015-221783 A (MARUHO CO., LTD.) 10.12.2015 (2015-12-10) claims | 1-10 |
| A | JP 2008-512214 A (CORIUM INTERNATIONAL, INC.) 24.04.2008 (2008-04-24) claims | 1-10 |
| A | US 2006/0193789 A1 (FOAMIX LTD.) 31.08.2006 (2006-08-31) paragraphs [0004], [0026], [0029], claims | 1-10 |
| A | JP 2002-536319 A (CIPLA LIMITED) 29.10.2002 (2002-10-29) claims | 1-10 |
| A | JP 2016-538311 A (GALDERMA SA) 08.12.2016 (2016-12-08) claims | 1-10 |

Form PCT/ISA/210 (continuation of second sheet) (January 2015)

**INTERNATIONAL SEARCH REPORT**
Information on patent family members

International application No.

PCT/JP2020/003947

| Patent Documents referred in the Report | Publication Date | Patent Family | Publication Date |
|---|---|---|---|
| JP 2015-521995 A | 03 Aug. 2015 | WO 2014/006201 A1 claims, page 7, page 10, lines 10-12, examples 1, 2 US 2015/0157729 A1 CN 104519869 A KR 10-2015-0027831 A | |
| JP 03-077820 A | 03 Apr. 1991 | (Family: none) | |
| JP 05-032535 A | 09 Feb. 1993 | (Family: none) | |
| JP 2004-067539 A | 04 Mar. 2004 | US 2005/0163831 A1 paragraph [0040] WO 2004/012734 A1 EP 1547595 A1 | |
| JP 2006-523662 A | 19 Oct. 2006 | US 2006/0223740 A1 paragraph [0002] WO 2004/091584 A1 KR 10-2006-0023114 A CN 1805733 A | |
| JP 2006-160610 A | 22 Jun. 2006 | US 2008/0124367 A1 paragraph [0005] WO 2006/059798 A1 EP 1839647 A1 KR 10-2007-0085587 A CN 101068529 A | |
| JP 2015-221783 A | 10 Dec. 2015 | (Family: none) | |
| JP 2008-512214 A | 24 Apr. 2008 | WO 02/087645 A1 claims US 2003/0170308 A1 CN 101018571 A KR 10-2007-0099533 A | |
| US 2006/0193789 A1 | 31 Aug. 2006 | EP 2422768 A2 claims | |
| JP 2002-536319 A | 29 Oct. 2002 | US 2004/0213744 A1 claims WO 00/045795 A2 | |
| JP 2016-538311 A | 08 Dec. 2016 | WO 2015/079016 A1 claims US 2017/0000812 A1 KR 10-2016-0082254 A CN 105916511 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011126796 A

- JP 2011126797 A

**Non-patent literature cited in the description**

- **ATSUSHI FUJITA.** *Pharmaceutical Bulletin,* 1954, vol. 2 (2), 163-173

- *Chemistry region,* 1957, vol. 11 (10), 719-725
- *Fragrance journal,* 1981, vol. 50, 79-82